(19) **Europäisches Patentamt**
**European Patent Office**
**Office européen des brevets**

(11) **EP 4 674 350 A1**

(12) **EUROPEAN PATENT APPLICATION**

(43) Date of publication:
**07.01.2026 Bulletin 2026/02**

(21) Application number: **24186732.4**

(22) Date of filing: **05.07.2024**

(51) International Patent Classification (IPC):
**A61B 6/00** (2024.01)

(52) Cooperative Patent Classification (CPC):
**A61B 6/545**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Koninklijke Philips N.V.**
**5656 AG Eindhoven (NL)**

(72) Inventors:
- **MAY, Jan Marek**
  **Eindhoven (NL)**
- **LUNDT, Bernd**
  **Eindhoven (NL)**
- **KOEPNICK, Johannes**
  **5656AG Eindhoven (NL)**

(74) Representative: **Philips Intellectual Property &
Standards**
**High Tech Campus 52**
**5656 AG Eindhoven (NL)**

(54) **METHOD AND DEVICE FOR PROSPECTIVE EFFECTIVE DOSE ESTIMATION**

(57) The invention concerns a method for estimating an effective dose applied to a subject being exposed to ionizing radiation during medical imaging, the method comprising the steps of obtaining image data of the subject arranged for the medical imaging using an image device, adapting a predetermined subject model comprising model subject information to the subject to be exposed to ionizing radiation by a processing unit on the basis of the image data of the subject; estimating the effective dose on the basis of the adapted predetermined subject model and based on received radiation source information with respect to the ionizing radiation the subject should be exposed to, wherein the effective dose is estimated for the current subject prior to the medical image acquisition. The invention further concerns a device for estimating an effective dose and a system comprising such a device.

Fig. 1

**Description**

FIELD OF THE INVENTION

The invention relates to the field of medical imaging, in particular X-ray imaging. More specifically, the invention relates to the field of estimating the effective dose of a subject prior to the medical image acquisition.

BACKGROUND OF THE INVENTION

A subject, such as a patient, which undergoes a medical image examination, is exposed to ionizing radiation. This radiation can be seen as a health risk, for example for developing cancer due to exposure of ionizing radiation. A relevant parameter for quantifying a stochastic health risk is the effective dose, as it accounts for the sensitivity of organs to ionizing radiation. The effective dose is measured in sievert and can be calculated using a known equation. For medical imaging tables with approximate effective radiation dose exist, which give information about the typical expected effective radiation dose for a specific examination. However, the effective dose varies from patient to patient and also depends on medical image parameters, such as collimation. Therefore, these tables are a rough approximation.

SUMMARY OF THE INVENTION

Therefore, there exists a need for optimizing the quality for estimating an effective dose for a subject undergoing medical imaging.
An object of the invention is to provide a method and a device for estimating an effective dose of a subject prior to the medical image acquisition, wherein the effective dose is personalized to the subject.
The object of the present invention is solved with the subject matter of the independent claims, wherein further embodiments are incorporated in the dependent claims.
According to a first aspect of the invention a method for estimating an effective dose of a subject being exposed to ionizing radiation during medical imaging is provided. The method comprising the steps of obtaining image data of the subject arranged for the medical imaging using an image device, adapting a predetermined subject model comprising model subject information to the subject to be exposed to ionizing radiation by a processing unit on the basis of the image data of the subject; estimating the effective dose on the basis of the adapted predetermined subject model and based on received radiation source information with respect to the ionizing radiation the subject should be exposed to, wherein the effective dose is estimated for the current subject prior to a medical image acquisition.
In the context of the present invention, the term "effective dose" shall be understood to describe a dose of ionizing radiation considering the sensitivity of specific organs for ionizing radiation, in particular X-ray radiation. The effective dose may be one of the important metrics regarding health effects of ionizing radiation on a subject, such as the human body. The effective dose may be measured in sievert. The effective dose enables the computation of a stochastic health risk. The term effective dose described herein may describe the local effective dose estimated for a part of the subject, for example for a specific organ of the human body. On the other hand, the term effective dose may be understood to describe the effective dose of the whole subject. The effective dose which is measured in sievert is computed as:

$$\tilde{E} = \sum_T W_T \sum_R W_R \frac{\int_T D_R(x, y, z) \rho(x, y, z) dV}{\int_T \rho(x, y, z) dV},$$

$$(1)$$

where $\tilde{E}$, is the effective dose in sievert applied to the entire organism, $W_T$ are the tissue weighting factors and $W_R$ are the radiation weighting factors. The absorbed dose in tissue $T$ by radiation type $R$ is given by $D_R(x, y, z)$ and $\rho(x, y, z)$ is the density at the location $x, y, z$. For the target application of X-ray imaging $W_R = 1$, the equation can be simplified to

$$\tilde{E} = \sum_T W_T \frac{\int_T D_R(x, y, z) \rho(x, y, z) dV}{\int_T \rho(x, y, z) dV}.$$

$$(2)$$

This equation allows to compute the effective dose applied to the human body using X-rays.

In the context of the present invention, the term "subject" shall be understood to describe for example a person which should be examined or only a part of the person. The method is not limited to human bodies, it may also apply to animal bodies. Preferably, the term subject may refer to a patient, but may not be limited to it. The method may be applied to animal examinations, hence may not be limited to human patients, and may also be applicable to parts of a patient or other subjects for which the effective dose should be estimated prior to an examination.

In the context of the present invention, the term "medical image acquisition" shall be understood to describe any examination, i.e., medical imaging, performed on a subject, for example on a patient, which uses ionizing radiation. For example, the medical imaging may be at least one of X-ray imaging, such as for example radiography, or fluoroscopy.

In the context of the present invention, the term "ionizing radiation" shall be understood to describe any type of radiation which may cause health risk for a subject for instance ionizing radiation in the context as described herein may be X-ray radiation.

In the context of the present invention, the term "radiation source information" shall be understood to describe any information derived from the radiation source, such as an X-ray tube. The information may be any parameter used for a setup of the radiation source, wherein the radiation source information comprises at least one of a source-image-distance (SID), a source voltage (kV), an emitted radiation energy (mAs), and collimation. In practice more than one of these parameters is set for a medical image acquisition. In particular, all parameters may be required to be set for the medical image acquisition. More in particular, for simulating the subject model the energy, i.e., the source voltage kV and the emitted radiation energy mAs should be known, for simulating the model properly.

In the context of the present invention, the term "subject model" shall be understood to describe a computer-generated model, such as for instance a statistical model, an image model, which both may be in particular a 3D model. The model may be generated from a simulation. On the other hand, the model may be obtained from previous medical images. Further, the subject model may be a standardized model of a specific subject type, wherein for different subject types different subject models exist. For example, the subject types may differ in size, age, weight, height, and gender. Furthermore, the subject model may be derived from different medical images received from different medial image systems, which allows to determine subject information.

In the context of the present invention, the term "model subject information" shall be understood to describe information contained in the predetermined subject model, wherein the model subject information comprises information about an organ position, organ sensitivity, which may be described with an organ (tissue) specific weighting factor. The weighting factor is a relative measure of the risk of stochastic effects that might result from irradiation of that specific organ (tissue). It depends on the variable sensitivity of organs and tissues of the subject to ionizing radiation.

In the context of the present invention, the term "image data" shall be understood to describe any data obtained by an image device, wherein the image device is configured to measure and/or receive subject information. Differently speaking, the image data may be obtained from an optical sensor and/or electronical sensor, in particular a 3D sensor, for example a depth camera. Accordingly, the image data may contain information about the surface of the subject.

In other words, the method may be used for estimating an effective dose of a subject, before the subject undergoes medical imaging, in particular prior to the medical image acquisition. The subject is not exposed to ionizing radiation during performing the method. Instead, the ionizing radiation may be simulated for the method based on the radiation source information, which will be described with the exemplary embodiments herein. Differently speaking, a simulation of ionizing radiation applied to a subject by estimating the effective dose applied to the subject is described. Accordingly, an estimation of the effective dose may be calculated before the subject has to be exposed. This allows an adaption of the effective dose by changing the parameter of the medical imaging system to reduce the exposure to ionizing radiation for the subject. The effective dose for determining a health risk of a subject, or body parts of the subject can be estimated by using a predetermined subject model which is adapted to the current subject. The obtained image data is used for the adaption of the predetermined subject model to the current subject, wherein for the estimation of the effective dose the adapted (predetermined) subject model is used. The processing unit may be able to perform any method step described therein. Further, the processing unit may be one processor or a processor assembly, wherein the processing unit may not be limited to this specific embodiment. Any processing device able to perform the herein described method may be used. A method and device may be provided for prospective estimation of the expected effective dose based on image data. This may have the advantage of estimating, or calculating, the effective dose at system level. This allows a user of the method, for example applied in a computer of a system or device, to estimate the effect of the current image source parameter, such as a collimation, on the effective dose and hence on the statistical health risk for the current subject, the current patient. This may help a user to reduce the effective dose applied to patients due to the estimation prior to the real image process and enables a smooth integration into the workflow without interruption. Furthermore, a biological effect of the image acquisition can be described and not only the technical effect, which helps to monitor and/or to specify the health risk of the subject such as a patient.

It should be noted that any feature, function, and/or element described in the following with reference to the method equally applies to the device, and vice versa. Accordingly, any feature, function, step, and/or element described in the following with reference to one aspect of the present disclosure equally applies to any other aspect of the present disclosure.

According to an exemplary embodiment of the invention, the image device may comprise at least a depth sensor. The method may further comprise the step of obtaining depth image data from the subject using the depth sensor, wherein the depth image data comprises at least one of a contour of the subject, a surface profile of the subject, a spatial profile of the subject, a 2D or 3D contour of the subject. When using a depth sensor, the step of adapting the predetermined subject model may further comprise the adaption of the predetermined subject model on the basis of the depth image data.

The image device may use only one depth sensor, which is able to determine for example surface information of the subject, which can be used to adapt the predetermined subject model. The step of adapting the predetermined model, when using the depth sensor, may be carried out by using the depth image data of the subject for the adaption.

It should be noted that the image device may not be limited to the specific depth sensor, this depth sensor is just an exemplary embodiment for a sensor for obtaining image data. Another sensor may also be possible, such as a 3D sensor. A 3D sensor applicable in the image device may be at least one of the following: an optical sensor, for example a laser, structured light sensors (RGB-D camera); ultrasonic sensor such as ultrasonic range finder; a Lidar sensor; an infrared sensor, such as a 3D TOF camera (TOF-time of flight).

According to an exemplary embodiment of the invention, the image device may further comprise an image sensor. The method may further comprise, when using the image sensor, obtaining image data from the subject using the image sensor; wherein the image data comprises a RGB image. When using the image sensor, the step of adapting the predetermined subject model may further comprise the adaption of the predetermined surface model on the basis of the image data.

The image device may comprise the image sensor additionally to the depth sensor, such that the image device comprises at least two sensors (depth and image). On the other hand, the image device may use at least two image sensors, such that no depth sensor is used. The two image sensors may be able to obtain image data from which surface information of the subject may be derived.

According to an exemplary embodiment of the invention, the method may further comprise the step of simulating the ionizing radiation emitted towards the subject for generating a path of rays, wherein the step of estimating the effective dose further uses the generated path of rays for estimating the effective dose. The simulation may be a mathematical simulation in the processing unit. The simulation may not necessarily be visible to a user when performing the simulation. The ionizing radiation may be divided into a plurality of rays, wherein for each ray a path from the radiation source to the radiation detector through the subject is determined. Together with the radiation source information and the path of the rays the effective dose may be estimated for each ray which reaches the patient.

According to an exemplary embodiment of the invention, the method may further comprise the steps of dividing the ionizing radiation to be emitted towards the subject into a plurality of rays of ionizing radiation, wherein the adapted predetermined subject model comprises a plurality of voxels. The method further comprises for one or more rays of ionizing radiation, assigning to at least one ray each voxel intersecting with the at least one ray in the adapted predetermined subject model for determining an absorbed radiation dose, wherein the step of estimating the effective dose comprises estimating the effective dose based on the absorbed radiation dose of each voxel intersecting with the at least one ray.

For example, one or more rays of ionizing radiation may pass through a voxel of the predetermined subject model, preferably to each voxel one ray may be assigned. But depending on the resolution of the adapted subject model also more than one voxel may be assigned to one ray. The number of voxels specifies the number of rays, wherein the number of voxels may be defined by the resolution of the subject model. For example, for a CT image a voxel may have an edge length smaller than one millimeter. For each voxel, the adapted predetermined subject model may be analyzed. For instance, to which part of the adapted subject model the voxel is assigned to and the amount of the absorbed radiation dose in this voxel. Depending on the weighting factor for the voxel, which can be determined due to the assignment of the single voxel to a position in the adapted subject model, the sensitivity can be determined and accordingly the estimated effective dose may be estimated. In particular, to one ray more than one voxel may be assigned, which means the ray intersects more than one voxel when passing through the subject model. For estimating the effective dose, the information from all voxels intersecting with the one ray may be used. The subject model, which means the predetermined and also the adapted subject model may be described as a 3D model which is divided into, in the sense of comprise, a plurality of voxels. Each voxel can be assigned to an organ in the subject model. Therefore, the effective dose may be estimated based on the above described procedure.

According to an exemplary embodiment of the invention, for each ray of the ionizing radiation one or more respective 3D coordinate may be associated to a position along the ray in the adapted predetermined subject model, wherein each position of the adapted predetermined subject model may be associated with a weighting factor. This may allow to assign to each 3D coordinate, wherein one 3D coordinate may be a voxel, along the ray when passing through the subject, a respective tissue (organ) weighting factor indicating the sensitivity of the organ. This may be used for the estimation of the effective dose. The ray of ionizing radiation passes through the (adapted) predetermined subject model and by passing through the model different 3D coordinates may be intersect. This means, that the ray may pass through more than one 3D coordinate having a specific weighting factor.

According to an exemplary embodiment of the invention, the method may further comprise an adaption of radiation parameters depending on the result of the estimated effective dose. For example, a collimation of parts, or the subject, to be exposed may be adapted, if the estimated dose would reach a threshold of causing a health risk. Hence, after performing the method of estimating the effective dose, the results of this estimation may be used for adapting the medical imaging process for the real exposure of the subject. On the other hand, the estimated effective dose may be collected and/or stored for comparison with future imaging processes on the same subject. Furthermore, the result of the effective dose estimation may be used for providing instructions for acting of the user or the subject. For example, a lead apron may be used for specific organs or body parts of the subject for preventing an effective dose causing a health risk.

According to an exemplary embodiment of the invention, the step of adapting the predetermined subject model may further comprises comparing the model subject information of the predetermined subject model with subject information from the subject to be exposed for determining the best matching predetermined subject model.

The adaption of the predetermined subject model may comprise using a plurality of predetermined subject models for comparison with the current subject. For example, the model subject information may comprise information about the gender, the age, the size, or the weight of the subject for finding the best matching predetermined subject model.

According to an exemplary embodiment of the invention, the step of adapting the predetermined subject model may be performed by using machine learning algorithm, in particular by using a convolutional neural network. An input for the training of the machine learning algorithm may be a plurality of predetermined subject models and a plurality of model subject information associated with each predetermined subject model and the obtained image data from the predetermined subject models.

The plurality of predetermined subject models may be received from an external device or system. In detail, images for creating the plurality of predetermined subject models may be received for example from other medical image devices, wherein these models may be based on images of different subjects, such as different patients. The associated model subject information may be for example a sensitivity of the subject, in particular of different organs of the subject, and a position of body parts of the subjects, in particular a position of the organs of the subject in the predetermined subject model. Based on the obtained image data from the image device the machine learning algorithm is able to find the best matching predetermined subject model out of the plurality of predetermined subject models. Also, other subject information, such as gender, size, weight, and age may be used for finding the best matching predetermined subject model.

According to an exemplary embodiment of the invention, one or more machine learning algorithm may perform the step of determining the predetermined subject model matching the subject to be exposed and the step of determining model transformation parameters for adapting the predetermined subject model to the subject to be exposed.

In other words, one machine learning algorithm may be used to find the best matching predetermined subject model for the current subject, and another machine learning algorithm may be used for defining model transformation parameters, which may be used for adapting to the current subject. Hence, for each step a different machine learning algorithm may be applied. The determination of the model transformation parameter may use registration techniques. Based on sensor data such as for example a depth image or RGB image, the predetermined subject model may be registered in 3D space. For determining the predetermined subject model matching the subject to be exposed not necessarily a machine learning algorithm must be used. Other techniques, such as look up tables may be sufficient.

According to an exemplary embodiment of the invention, the step of adapting the predetermined subject model may further comprises: receiving the predetermined subject model, in particular derived from CT or MRT scans provided to the processing unit, receiving subject information from the image device and/or an external information device, wherein the subject information is at least one of a subject size, subject gender, subject weight, adapting the predetermined subject model to the subject on the basis of the received subject information.

The subject information may be revived from the image device. For instance, from the image data of the image device it may be determined a size, a weight, or a gender of the patient. On the other hand, the subject information may be received from an external source, such as a hospital information system, wherein patient specific information are stored. For predetermining a model, at least gender, weight and size may be needed. The age of the subject may be optional. With this information a selection could be done using conventional algorithms to select the best predetermined subject model. Using depth information, a better preselection could be possible. In this case a machine learning algorithm may be used. The adaption of the model to the subject may use at least the depth data.

According to an exemplary embodiment of the invention, the step of estimating the effective dose may further comprise at least one of the following steps: determining at least one voxel assigned to an organ of the subject using the adapted predetermined subject model; weighting the absorbed radiation dose with an organ specific weighting factor, which is received from the predetermined subject model; integrating the weighted absorbed radiation dose for each ray intersecting the organ of the subject; wherein estimating the effective dose of the organ of the subject is based on the integrated weighted absorbed radiation dose of each ray intersecting the organ of the subject.

When determining a voxel assigned to an organ of the subject the effective dose may be estimated for this specific voxel. It may be applicable that a plurality of voxels is assigned to at least one organ of the subject using the predetermined subject model. Therefore, it may be applicable that a plurality of voxel is determined, which is assigned to one and the same organ. The number of voxels may depend on the (adapted) predetermines subject model, such that the plurality of voxels may be assigned to one organ of the subject, or for each organ of the subject at least one voxel may be assigned. With the weighting factors of the tissue (organ) used for weighting the absorbed radiation dose and integrating this weighted absorbed radiation dose the effective dose may be estimated.

According to an exemplary embodiment of the invention, the method may further comprise the step of visualizing the estimated effective dose on a user interface, wherein the step of visualizing comprises the overlay of the estimated effective dose on a live image of the subject. On the other hand, for the visualization a standard image illustrating a subject or part of the subject may be used, which may be a schematically illustration of the subject. Further, it may be possible to visualize the effective dose with an overlay on any suitable image for illustrating the effective dose, such as an image of the current subject or only schematically image illustrating a standard subject, or any combination thereof.

The estimated effective dose may be visualized as overlay on for example an RGB live camera image to highlight areas that are particularly exposed to radiation. For instance, the effective dose may be visualized as a local effective dose, which is for example an effective dose for each organ of a subject. On the other hand, the effective dose may be visualized as an overall effective dose of the complete subject. This may allow a user to directly inspect the effect of the current image parameter (set for the imaging, such as the system parameters of the source, i.e., X-ray tube) for example a collimation on the estimated effective dose and hence on the statistical health risk for the current subject. Such an overlay helps the user to reduce the effective dose applied to patients and enables a smooth integration into the workflow without interruptions.

The live image may be a current view of a camera image visualizing the estimated effective dose within a collimated area on the subject and/or the overall effective dose for the current view visualized on the user interface.

According to an exemplary embodiment of the invention, the visualized estimated effective dose may be displayed as a color overlay visualizing different strength of the effective dose. The visualized estimated effective dose may be a so-called heat map, wherein the color overlay is indicated with red and blue color. A red color visualizes areas of the subject, or for example regions of organs of the subject, which have a high effective dose and blue areas of the visualization indicate a lower estimated effective dose. Different color gradients from blue, to orange, to red may be used to indicate an intensity or strength distribution.

According to a second aspect of the invention, a device for estimating an effective dose of a subject being exposed to ionizing radiation during medical imaging is provided. The device comprising an image device configured to obtain image data of the subject arranged for the medical imaging, a processing unit configured to adapt a predetermined subject model comprising model subject information to the subject to be exposed to the ionizing radiation on the basis of the image data of the subject, estimate the effective dose of the subject on the basis of the adapted predetermined subject model and based on received radiation source information with respect to the ionizing radiation the subject should be exposed to, wherein the effective dose is estimated for a current subject prior to medical image acquisition.

The device for estimating the effective dose of the subject may use, for example by using the processing unit, the method as described for the different exemplary embodiments herein. Accordingly, the device may comprise at least one of a depth sensor, an image sensor, two image sensors, as described through the exemplary embodiments of the method. Wherein the device may be configured for adapting the predetermined subject model on the basis of the image data received from those different sensors. The device may be further configured for dividing the ionizing radiation to be emitted towards the subject into a plurality of rays of ionizing radiation, dividing the adapted predetermined subject model into a plurality of voxels, assigning the one or more rays to one or more specific voxel(s) along the ray in the adapted predetermined subject model for determining an absorbed radiation dose, estimating the effective dose based on the absorbed radiation dose of the specific voxel. Further, the device may be configured to apply a machine learning algorithm such as the herein described convolutional neural network.

According to a third aspect of the invention, a system for estimating an effective dose of a subject being exposed to ionizing radiation during medical imaging is described, the system comprising a medical imaging device comprising a radiation source emitting ionizing radiation towards the subject, wherein the subject is arranged between the radiation source and a radiation detector, and a device according to any of the herein described embodiments.

According to a fourth aspect of the invention, a computer program comprising instructions is described, which, when the program is executed by a computer, causes the computer to carry out the method of any one of the embodiments as described herein above. In detail, the program may cause the computer to carry out the method of estimating an effective dose of a subject being exposed to ionizing radiation during medical imaging as described with any one of the embodiments herein. Further, the program may additionally and/or separately cause the computer to carry out the, for instance computer-implemented, method for visualizing the estimated effective dose as described according any of the embodiments herein.

The computer program element may be part of a computer program, but it can also be an entire program by itself. For example, the computer program element may be used to update an already existing computer program to get to the present invention.

6

The program element may be stored on a computer readable medium. The computer readable medium may be seen as a storage medium, such as for example, a USB stick, a CD, a DVD, a data storage device, a hard disk, or any other medium on which a program element as described above can be stored.

According to a fifth aspect of the invention, A computer-readable medium comprising instructions which, when executed by a computer, cause the computer to carry out the method of any one of the embodiments as described herein. In detail, the computer-readable medium may cause the computer to carry out the method for estimating an effective dose of a subject being exposed to ionizing radiation during medical imaging as described with any one of the embodiments herein. Further, the computer-readable medium may additionally and/or separately cause the computer to carry out the computer-implemented method for visualizing the estimated effective dose as described with any of the embodiments.

It has to be noted that embodiments of the invention have been described with reference to different subject matters. In particular, some embodiments have been described with reference to apparatus type claims whereas other embodiments have been described with reference to method type claims. However, a person skilled in the art will gather from the above and the following description that, unless other notified, in addition to any combination of features belonging to one type of subject matter also any combination between features relating to different subject matters, in particular between features of the apparatus/device type claims and features of the method type claims is considered as to be disclosed with this application.

It should be noted that the term "comprising" does not exclude other elements or steps and "a" or "an" does not exclude a plurality. Also, elements described in association with different embodiments may be combined. It should also be noted that reference signs in the claims should not be construed as limiting the scope of the claims.

BRIEF DESCRIPTION OF THE DRAWINGS

The aspects defined above, and further aspects of the present invention are apparent from the examples of embodiment to be described hereinafter and are explained with reference to the examples of embodiment. The invention will be described in more detail hereinafter with reference to examples of embodiment but to which the invention is not limited.

Fig 1 illustrates an arrangement for a device for estimating an effective dose of the subject exposed to ionizing radiation during medical imaging according to an exemplary embodiment of the invention.

Fig. 2 shows a schematical flow diagram illustrating a method for estimating an effective dose of the subject to be exposed to ionizing radiation according to an exemplary embodiment of the invention.

Fig. 3 illustrates a visualization of the estimated effective dose according to an exemplary embodiment of the invention.

DETAILED DESCRIPTION OF EMBODIMENTS

The illustrations in the drawings are schematic. It is noted that in different Figures similar or identical elements are provided with the same reference signs.

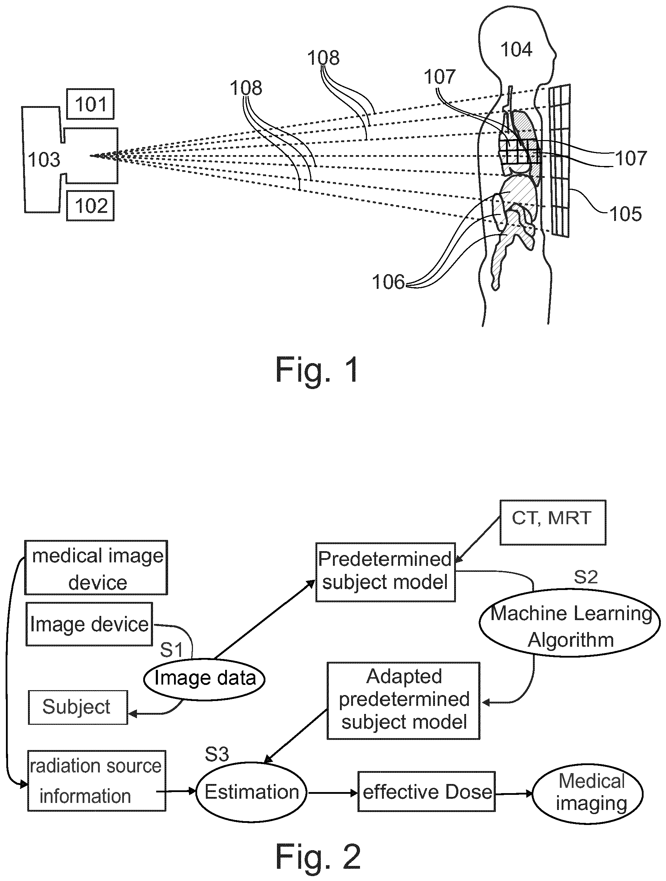

Fig. 1 illustrates an arrangement for a device and/or system for estimating an effective dose of a subject 104 exposed to ionizing radiation 108 during medical imaging according to an exemplary embodiment of the invention. In particular, Fig. 1 illustrates a simulation of the rays 108 emitted from the device, which may be the (simulated) radiation source of a medical image device 103, to the radiation detector 105 and through the subject 104. The device may be configured to perform the method for estimating an effective dose of a subject 104, before the subject 104 undergoes medical imaging. The subject 104 is not exposed to ionizing radiation 108 during performing the method. The image data is obtained by the image device 101 and 102, wherein the image device according to this embodiment comprises two sensors 101 and 102. It should be noted that the image device comprises not necessarily two devices 101, 102. One image device, such as a depth camera may be sufficient. For example, 101 may be the depth sensor and 102 may be the image sensor. The obtained image data from the image device 101, 102 is used for the adaption of the predetermined subject model to the current subject, wherein for the estimation of the effective dose the adapted (predetermined) subject model is used. When using for example just one depth sensor 101, the method may comprise the step of obtaining depth image data from the subject 104 using the depth sensor 101, wherein the depth image data comprises at least one of a contour of the subject (as illustrated in Fig. 1), a surface profile of the subject, a spatial profile of the subject, a 2D or 3D contour of the subject. For instance, if the image device may further comprise and use a second image sensor 102. If the image sensor 102 is used, the method may further comprise, obtaining image data from the subject 104 using the image sensor; wherein the image data comprises a RGB image. Further, if the image sensor 102 is used, the step of adapting the predetermined subject model may further comprises the adaption of the predetermined surface model on the basis of the image data.

In Fig. 1 the radiation source of the medical image device 103 emits the ionizing radiation, which is illustrated as a plurality of rays 108 towards the subject 104. With Fig. 1 also the step of dividing the ionizing radiation to be emitted towards the subject 104 into a plurality of rays of ionizing radiation 108, and that the adapted predetermined subject model comprises a plurality of voxels 107 is illustrated. It can be seen from Fig. 1, that each ray 108 may be assigned to one or more specific voxel 107, further, that each voxel 107 can be assigned to a position at the subject 104 (which can also be seen as an illustration of the adapted predetermined subject model). A single ray 108 may pass through more than voxel. For each voxel 107 intersecting with one single ray 108 the absorbed dose can be calculated and with the weighting factors determined from the position of the voxel 107 in the subject model 104, the effective dose can be estimated. As can be seen in Fig. 1 one ray 108 may intersect with voxels 107 from different organs.

Fig. 2 shows a schematic flow diagram illustrating a method for estimating an effective dose of the subject to be exposed to ionizing radiation according to an exemplary embodiment of the invention. The method comprising step S1 of obtaining image data of the subject arranged for the medical imaging using an image device, step S2 adapting a predetermined subject model comprising model subject information to the subject to be exposed to ionizing radiation by a processing unit on the basis of the image data of the subject; step S3 estimating the effective dose on the basis of the adapted predetermined subject model and based on received radiation source information with respect to the ionizing radiation the subject should be exposed to, wherein the effective dose is estimated for the current subject prior to the medical image acquisition. Before the method may be performed, a positioning of the patient may be necessary and that the elements of the medial image device, such as for example the tube, detector, collimator, or table on which the subject is positioned need to be arranged. These may be additional steps prior to step S1. For step S2 additional steps such as a step of providing the predetermined subject model (by the processor) and a step of applying a machine learning algorithm may be performed. A set of subject models is provided, derived from CT, MR, or other suitable image data, as an input for predetermining the best matching subject model and for adapting the predetermined subject model in step S2 to the subject 104. The adaption of the predetermined subject model may be performed in the step of applying the machine learning algorithm. After the effective dose is estimated in step S3, the effective dose which the subject would be exposed to is determined. After performing the method, the medical imaging may be performed, which is indicated in Fig. 2 with the step after the estimation in step S3.

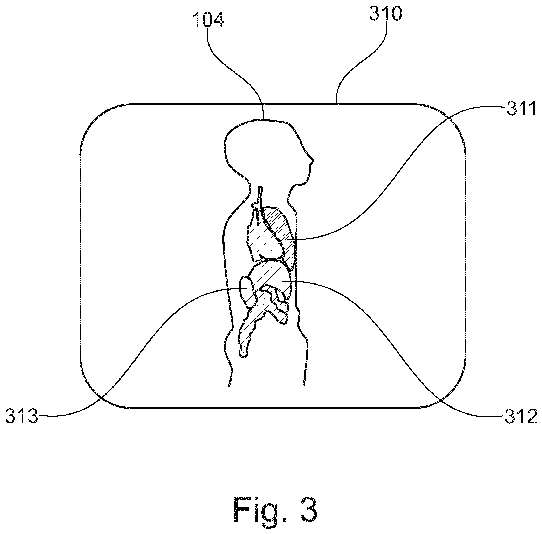

Fig. 3 illustrates a visualization of the estimated effective dose according to an exemplary embodiment of the invention. The method may comprise the step of visualizing the estimated effective dose on a user interface 310, wherein the step of visualizing comprises the overlay of the estimated effective dose on a live image of the subject 104. In Fig. 3 the subject 104 may be displayed on the user interface 310 as a contour. Other visualization of the subject 104 may be possible, for example also a live image or video of the exact subject in the sense of a photographical illustration may be possible. The estimated effective dose is displayed in Fig. 3 for different organs of the subject 104. For example, the lung 311 comprises a darker shadow (in reality it may be illustrated in a dark red color) than the liver 312 or the kidney 313. The kidney 313 is illustrated with a light gray shadow, which may be shown on the user interface 310 in reality with a blue color representation.

As described above, the visualized estimated effective dose for the different organs 311, 312, 313 (and also the other not numbered organs) may be displayed as a color overlay, such as a heat map, visualizing different strengths of the effective dose. The visualized estimated effective dose may be a so-called heat map, wherein the color overlay is indicated with red and blue color. A red color visualizes areas of the subject, or for example regions of organs of the subject, which have a high effective dose, the lung 311, and blue areas of the visualization indicate a lower estimated effective dose, the kidney 313 in Fig. 3. Different color gradients from blue, to orange, to red may be used to indicate an intensity or strength distribution. In Fig. 3 the different color distributions are indicated with different gray shades.

LIST OF REFERENCE SIGNS:

| | |
|---|---|
| 101 | image device, depth sensor |
| 102 | image device, RGB sensor |
| 103 | medical image device |
| 104 | subject |
| 105 | detector |
| 106 | organ |
| 107 | voxel |
| 108 | rays of ionizing radiation |
| 310 | interface |
| 311 | lung |
| 312 | liver |
| 313 | kidney |

**Claims**

1. Method for estimating an effective dose of a subject being exposed to ionizing radiation during medical imaging, the method comprising the steps of

   obtaining image data of the subject arranged for the medical imaging using an image device;
   adapting a predetermined subject model comprising model subject information to the subject to be exposed to ionizing radiation by a processing unit on the basis of the image data of the subject;
   estimating the effective dose on the basis of the adapted predetermined subject model and based on received radiation source information with respect to the ionizing radiation the subject should be exposed to.
   wherein the effective dose is estimated for the current subject prior to a medical image acquisition.

2. The method according to claim 1,

   wherein the image device comprises at least a depth sensor, wherein the method further comprises
   obtaining depth image data from the subject using the depth sensor, wherein the depth image data comprises at least one of a contour of the subject, a surface profile of the subject, a spatial profile of the subject, a 2D or 3D contour of the subject;
   wherein the step of adapting the predetermined subject model further comprises the adaption of the predetermined subject model on the basis of the depth image data.

3. The method according to claim 2,

   wherein the image device further comprises an image sensor, wherein the method further comprises
   obtaining image data from the subject using the image sensor; wherein the image data comprises a RGB image, wherein the step of adapting the predetermined subject model further comprises the adaption of the predetermined surface model on the basis of the image data.

4. The method according to any of the preceding claims, wherein the method further comprises the step of

   simulating the ionizing radiation emitted towards the subject for generating a path of rays,
   wherein the step of estimating the effective dose further uses the generated path of rays for estimating the effective dose.

5. The method according to any of the preceding claims, wherein the method further comprises the steps of

   dividing the ionizing radiation to be emitted towards the subject into a plurality of rays of ionizing radiation,
   wherein the adapted predetermined subject model comprises a plurality of voxels,
   for one or more rays of ionizing radiation assigning to at least one ray each voxel intersecting with the at least one ray in the adapted predetermined subject model for determining an absorbed radiation dose,
   wherein the step of estimating the effective dose comprises estimating the effective dose based on the absorbed radiation dose of each voxel intersecting with the at least one ray.

6. The method according to any of the preceding claims,
   wherein the step of adapting the predetermined subject model further comprises comparing the model subject information of the predetermined subject model with subject information from the subject to be exposed for determining the best matching predetermined subject model.

7. The method according to any of the preceding claims,

   wherein the step of adapting the predetermined subject model is performed by using machine learning algorithm, in particular by using a convolutional neural network;
   wherein an input for the machine learning algorithm is a plurality of predetermined subject models and a plurality of model subject information associated with each predetermined subject model and the obtained image data from the predetermined subject models.

8. The method according to claim 7,

wherein the one or more machine learning algorithm is configured to perform
determining the predetermined subject model matching the subject to be exposed and
determining model transformation parameters for adapting the predetermined subject model to the subject to be exposed.

9. The method according to any of the preceding claims,

   wherein the step of adapting the predetermined subject model further comprises
   receiving the predetermined subject model, in particular derived from CT or MRT scans provided to the processing unit,
   receiving subject information from the image device and/or an external information device, wherein the subject information is at least one of a subject size, subject gender, subject weight,
   adapting the predetermined subject model to the subject on the basis of the received subject information.

10. The method according to any of the preceding claims 4 to 9,
    wherein for each ray of the ionizing radiation one or more respective 3D coordinate can be associated to a position along the ray in the adapted predetermined subject model, wherein each position of the adapted predetermined subject model is associated with a weighting factor.

11. The method according to any of the preceding claims 5 to 10, wherein the step of estimating the effective dose further comprises

    determining at least one voxel assigned to an organ of the subject using the adapted predetermined subject model;
    weighting the absorbed radiation dose with an organ specific weighting factor, which is received from the predetermined subject model,
    integrating the weighted absorbed radiation dose for each ray intersecting the organ of the subject,
    wherein the estimating the effective dose of the organ of the subject is based on the integrated weighted absorbed radiation dose of each ray intersecting the organ of the subject.

12. The method according to any of the preceding claims, wherein the method further comprising the step of

    visualizing the estimated effective dose on a user interface,
    wherein the step of visualizing comprises the overlay of the estimated effective dose on a live image of the subject.

13. The method according to claim 12,
    wherein the visualized estimated effective dose is displayed as a color overlay visualizing different strength of the effective dose.

14. A device for estimating an effective dose of a subject being exposed to ionizing radiation during medical imaging, the device comprising

    an image device configured to obtain image data of the subject arranged for the medical imaging,
    a processing unit configured to
    adapt a predetermined subject model comprising model subject information to the subject to exposed to the ionizing radiation on the basis of the image data of the subject,
    estimate the effective dose of the subject on the basis of the adapted predetermined subject model and based on received radiation source information with respect to the ionizing radiation the subject should be exposed to,
    wherein the effective dose is estimated for a current subject prior to medical image acquisition.

15. A system for estimating an effective dose of a subject being exposed to ionizing radiation during medical imaging, the system comprising

    a medical imaging device comprising a radiation source emitting ionizing radiation towards the subject, wherein the subject is arranged between the radiation source and a radiation detector, and
    a device according to claim 14.

Fig. 1

Fig. 2

Fig. 3

Europäisches
Patentamt

European
Patent Office

Office européen
des brevets

# EUROPEAN SEARCH REPORT

**Application Number**

EP 24 18 6732

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 854 307 A1 (KONINKLIJKE PHILIPS NV [NL]) 28 July 2021 (2021-07-28) * paragraph [0022] - paragraph [0103] * ----- | 1-15 | INV. A61B6/00 |
| A | WO 2017/103238 A1 (KONINKLIJKE PHILIPS NV [NL]) 22 June 2017 (2017-06-22) * page 6, line 16 - page 12, line 15 * ----- | 1-15 | |
| A | US 2017/245825 A1 (STAR-LACK JOSH [US] ET AL) 31 August 2017 (2017-08-31) * paragraph [0015] - paragraph [0065] * ----- | 1-15 | |
| A | EP 3 639 894 A1 (VARIAN MEDICAL SYSTEMS INT AG [CH]) 22 April 2020 (2020-04-22) * paragraph [0041] - paragraph [0118] * ----- | 1-15 | |

**TECHNICAL FIELDS SEARCHED (IPC)**

A61B

The present search report has been drawn up for all claims

| Place of search | Date of completion of the search | Examiner |
|---|---|---|
| Munich | 27 November 2024 | Hooper, Martin |

EPO FORM 1503 03.82 (P04C01)

## ANNEX TO THE EUROPEAN SEARCH REPORT
## ON EUROPEAN PATENT APPLICATION NO.

EP 24 18 6732

27-11-2024

| Patent document cited in search report | | | Publication date | Patent family member(s) | | Publication date |
|---|---|---|---|---|---|---|
| EP 3854307 | A1 | 28-07-2021 | NONE | | | |
| WO 2017103238 | A1 | 22-06-2017 | CN | 108471996 A | 31-08-2018 |
| | | | | EP | 3389494 A1 | 24-10-2018 |
| | | | | US | 2018368785 A1 | 27-12-2018 |
| | | | | WO | 2017103238 A1 | 22-06-2017 |
| US 2017245825 | A1 | 31-08-2017 | NONE | | | |
| EP 3639894 | A1 | 22-04-2020 | CN | 111068187 A | 28-04-2020 |
| | | | | EP | 3639894 A1 | 22-04-2020 |
| | | | | US | 2020121951 A1 | 23-04-2020 |
| | | | | US | 2020406062 A1 | 31-12-2020 |
| | | | | US | 2021402215 A1 | 30-12-2021 |
| | | | | US | 2023158335 A1 | 25-05-2023 |
| | | | | US | 2024226604 A1 | 11-07-2024 |